# EUROPEAN PATENT SPECIFICATION

(11) **EP 2 157 947 B1**
(45) Date of publication and mention of the grant of the patent: **10.07.2013**
(21) Application number: 08755651.0
(22) Date of filing: 16.05.2008
(51) Int. Cl.: A61F 13/00

(54) **WATER RESISTANT ELASTICIZED RETENTION BANDAGE AND UNDERCAST LINER**
WASSERFESTER ELASTISCHER HALTEVERBAND UND GIPSAUSKLEIDUNG
BANDAGE DE RÉTENTION ÉLASTIQUE RÉSISTANT À L'EAU ET REVÊTEMENT SOUS-PLÂTRE

(30) Priority: 17.05.2007 US 938505 P; 15.05.2008 US 121165
(43) Date of publication of application: 03.03.2010
(73) Proprietor: BSN Medical, Inc., Charlotte NC 28209 (US); Evans, John C., Lancashire OL16 3RF (GB)
(72) Inventor: The designation of the inventor has not yet been filed
(74) Representative: Misselbrook, Paul
(86) International application number: PCT/US2008/063841
(87) International publication number: WO 2008/144479

(56) References cited:
- WO-A-2005/052235
- WO-A-2007/051122
- US-A- 5 817 391
- US-A1- 2004 200 539

## Description

### Technical Field and Background of the Invention

The invention relates to water resistant medical bandages and more particularly to a water resistant elasticized bandage and undercast liner.

Current bandages are constructed from woven or knitted fabrics using natural or synthetic yarns. These yarns are difficult to dry once wet and generally have poor air permeability and limited water resistance. The bandage may be used to secure a splint, cast, padding, or swab against the skin for extended periods of time. Because of the poor air permeability of current bandages, this can lead to poor skin conditions such as maceration of the skin, bacteria growth, and odors.
W02007/051122 discloses an undercast liner in which the fabric has two opposing faces and an intermediate spacer area. The liner is constructed at least in part of hydrophobic, water resistant monofilament yarn.
W02005/052235 discloses a spacer fabric having cushioning properties comprising a first outer layer and second outer layer and an intermediate spacer layer integrated with the first and second outer layer. At least part of the one of the outer layers comprises hybride yarns.

Accordingly, there is a need for a bandage or cast liner that has sufficient porosity and strength so as to not cause skin maceration, bacteria growth, and odorous conditions.

### Summary of the Invention

Therefore, it is an object of the invention to provide a water-resistant elastic retention bandage.

It is another object of the invention to provide a water-resistant cast liner.

It is another object of the invention to provide a cast liner having improved bacterial prevention characteristics.

According to the present invention there is provided a fabric substrate suitable for lining between a cast and a person as set forth in the appended claims. Other features of the invention will be apparent from the dependent claims, and the description which follows.

### Brief Description of the Drawings

Some of the objects of the invention have been set forth above. Other objects and advantages of the invention will become apparent when the description thereof is taken in conjunction with the following drawings, in which:

Figure 1 is a perspective view of a roll of an undercast liner according to one embodiment of the invention;

Figure 2 is a view illustrating application of the undercast liner to the wrist and forearm;

Figures 3 and 4 illustrate preparation of a cast tape for application over the undercast liner;

Figures 5 and 6 illustrate application of the cast tape to the undercast liner;

Figure 7 illustrates the completed cast;

Figure 8 illustrates a liner in the form of a tubular sleeve;

Figure 9 shows the sleeve in place on an arm prior to application of a cast tape bandage;

Figure 10 is a perspective view of a water-resistant elasticized retention bandage according to a preferred embodiment of the invention;

Figure 11 is another perspective view of the retention bandage of Figure 10;

Figure 12 is yet another perspective view of the retention bandage of Figure 10;

Figures 13 and 14 illustrate the retention bandage of Figure 10 being used to secure a splint in position against the anatomy of a patient; and

Figure 14 illustrates the retention bandage in a rolled form.

### Description of the Preferred Embodiments and Best Mode

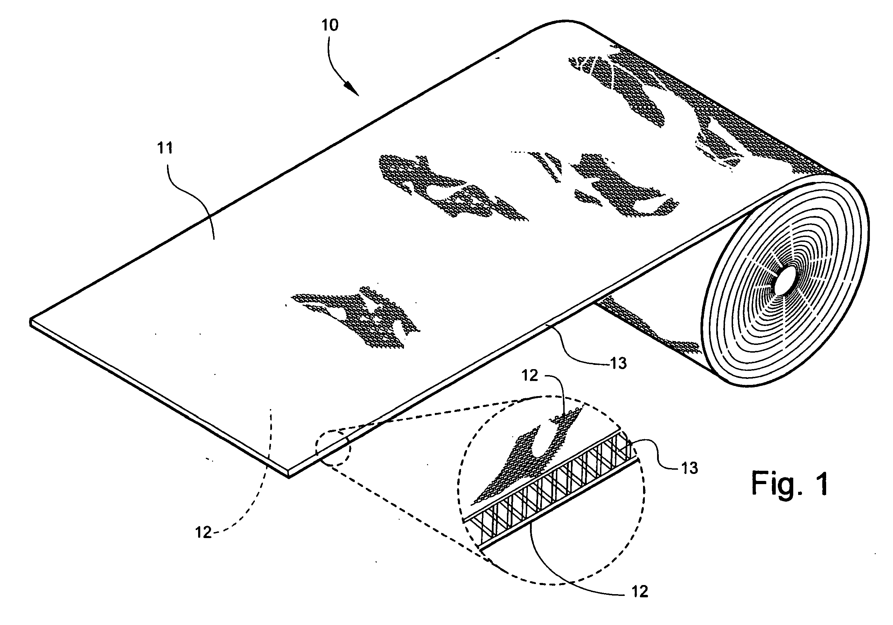

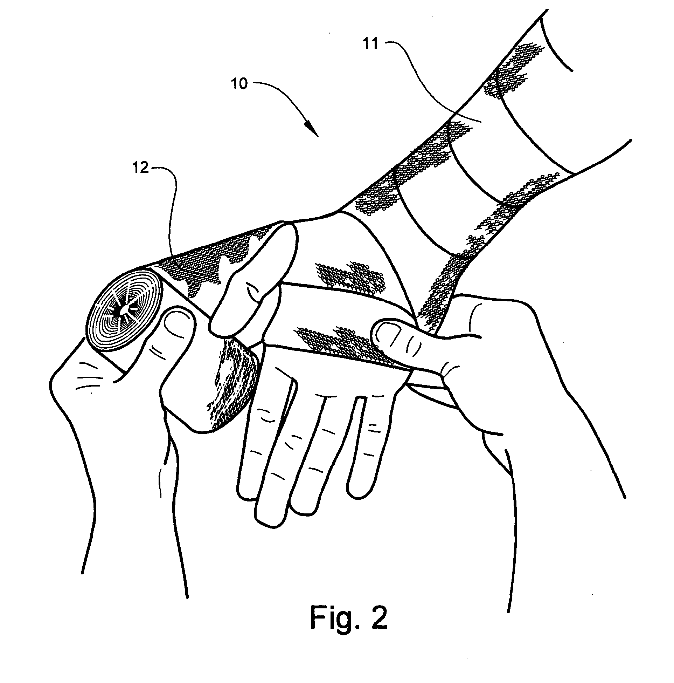

Referring now specifically to the drawings, a undercast liner according to the present invention is illustrated in Figure 1 and shown generally at reference numeral 10. The undercast liner 10 is easily dispensed from the roll, as shown in Figure 2. The liner 10 includes two opposing faces 11, 12 and an intermediate spacer area 13 that both separates and interconnects the faces 11, 12, as described in further detail below. The undercast liner 10 can be formed in a tubular form and formed in a roll.

The liner 10 can be constructed using any suitable organic or inorganic monofilament yarn, preferably a hydrophobic/water-resistant monofilament yarn such as polypropylene, polyester, polyethylene, and nylon. The monofilament yarn used for constructing the liner 10 preferably has a diameter of at least 0.03 mm. The liner 10 is constructed in a spacer fabric construction to provide sufficient cushioning and breathability. The use of a monofilament hydrophobic yarn on both faces 11, 12 and in the spacer area 13 provides enhanced water resistance, light weight, breathability, and resistance to collapse and degradation due to exposure to moisture and bacteria during extended use.

The liner 10 is formed using any suitable fabric forming technology such as weaving, various knitting techniques such as, for example, weft knitting and warp knitting, non-woven, stitching, or a combination of these techniques. Preferably, the structure should provide some stretch in both the length and width directions, and facilitate conforming the undercast liner 10 around an anatomical shape during application.

The liner 10 can be treated with one or more finishes to provide additional water resistance, anti-bacterial, anti-odor characteristics, or aromatherapy to improve the functionality or enhance the cast-wearing experience for the patient. Alternatively, the liner 10 can be fabricated from modified or treated monofilament yarns incorporating suitable fillers or finishes to improve the performance of the liner 10.

The liner 10 may also be provided with an adhesive coating on one or both faces 11, 12 to aid in application to the patient. The adhesive is preferably any suitable low tack, pressure sensitive adhesive, such as an acrylic or silicone adhesive. The adhesive aids in application by adhering to itself and thus maintaining the exact placement of the layers relative to each other as the liner 10 is applied by the cast technician.

In one embodiment, the liner 10 is constructed as a spacer fabric using polypropylene monofilament and a low tack, pressure sensitive adhesive on one surface. The monofilament yarn has a diameter of at least 0.03 mm, and preferably between about 0.05 and about 0.25 mm. Preferably, the liner 10 requires no additional finish or water repellency treatment.

More specifically, the liner 10 is constructed of a polypropylene monofilament yarn on a double needle bed knitting machine, and can be knitted on either a warp knitting Raschel machine or a Crochet knitting machine. The liner 10 is preferably constructed using a pillar and inlay stitch on the surfaces 11, 12 and a 3 or 5 needle V in the spacer area 13. The yarn has a diameter of between about 0.03 and about 0.25 mm. The fabric for the liner 10 is formed with at least about 50 courses per meter and preferably between about 200 and about 850 courses per meter. The liner 50 weighs between about 50 and about 400 grams per square meter, and more preferably between about 100 and about 250 grams per square meter. The liner 10 has a nominal thickness when not compressed or under tension of between about 1.5 and about 3.5 mm.

Alternatively, an undercast liner may be constructed as a spacer fabric with at least one of the yarns being a multifilament or spun yarn in order to provide even more patient comfort. The liner can be treated with a suitable fluorochemical, silicone, or other water repellant finish to improve drainage and provide faster drying.

Referring now to Figure 2, the undercast liner 10 is applied to the injured limb in a conventional manner. As noted above, the stretch provided by the undercast liner 10 permits a fast, accurate, closely-conforming application without wrinkles or creases.

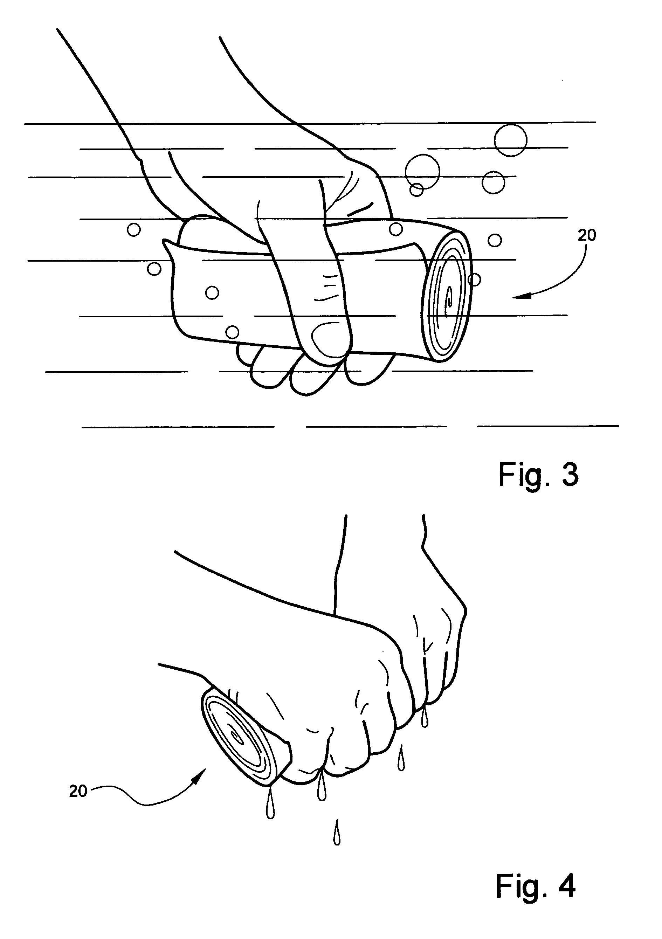

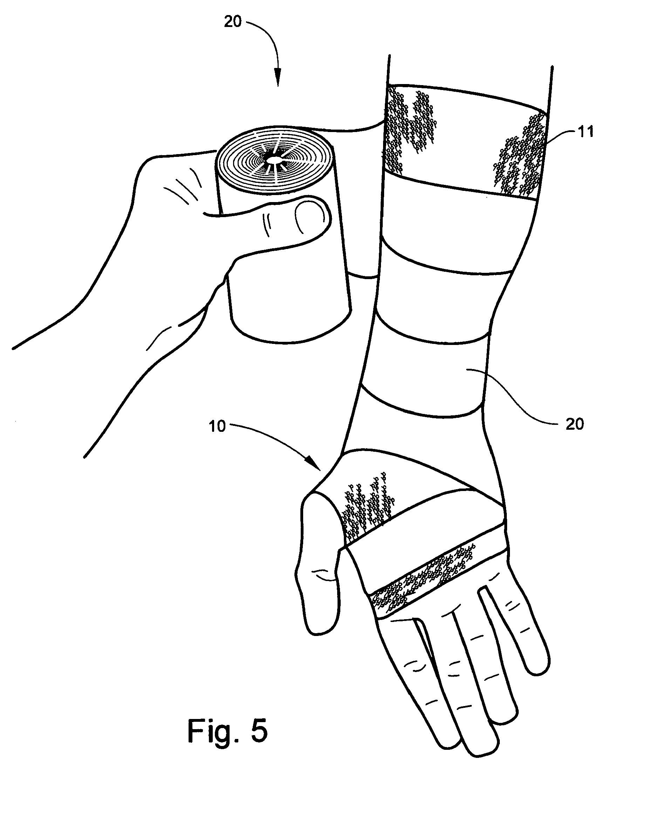

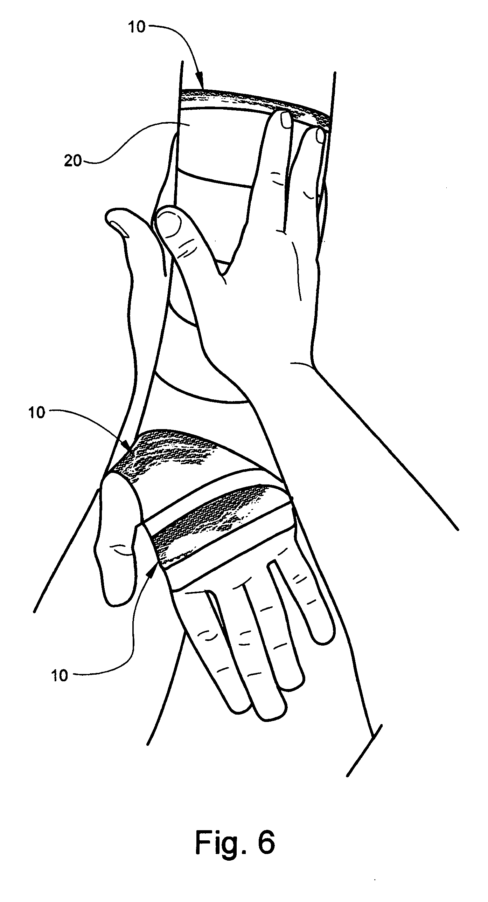

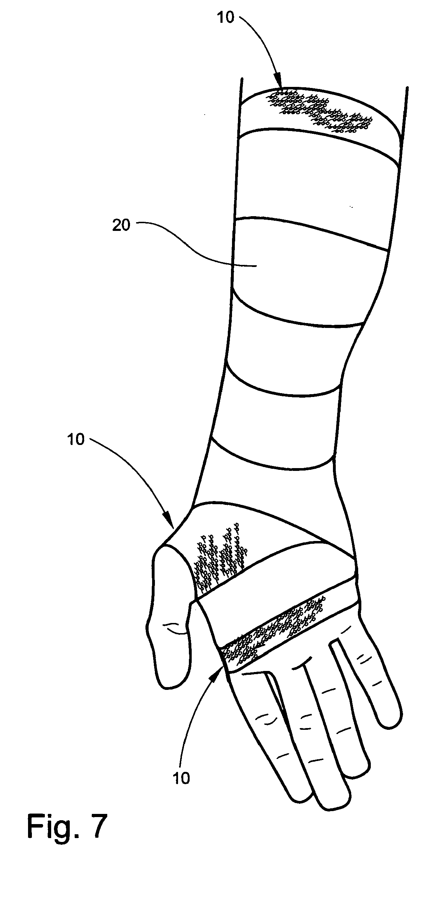

As is shown in Figures 3-7, after application of the undercast liner 10, a conventional cast tape 20 is wetted, Figure 3, excess water removed by wringing, Figure 4, and applied to the injured limb, Figures 5-7, taking care in the conventional manner to avoid overlapping the undercast liner 10 on opposite ends, leaving a short width of exposed undercast liner 10.

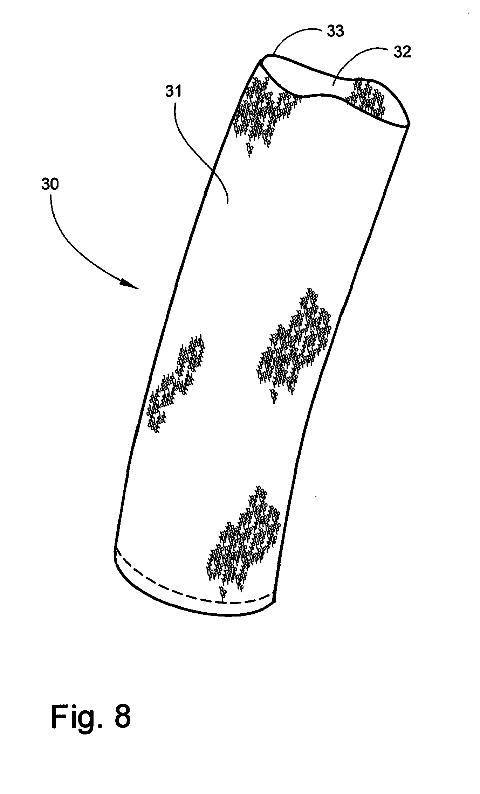

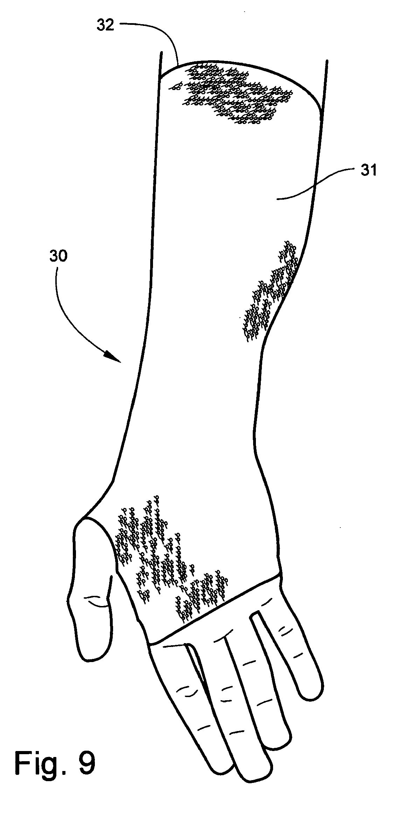

Referring now to Figure 8, a circular knit liner 30 is shown, preferably with the same constructions described above. The liner 30 includes an outer face 31, an inner face 32, and a spacer area 33. The spacer are 33 both separates and interconnects the two faces 31, 32, as shown in Figure 1. Instead of wrapping, the liner 30 is pulled onto the limb as shown in Figure 9, in the same manner as a conventional stockinette. Thereafter, a cast tape 20 is applied in a conventional manner.

A further embodiment includes a knitted spacer fabric constructed from monofilament yarns.

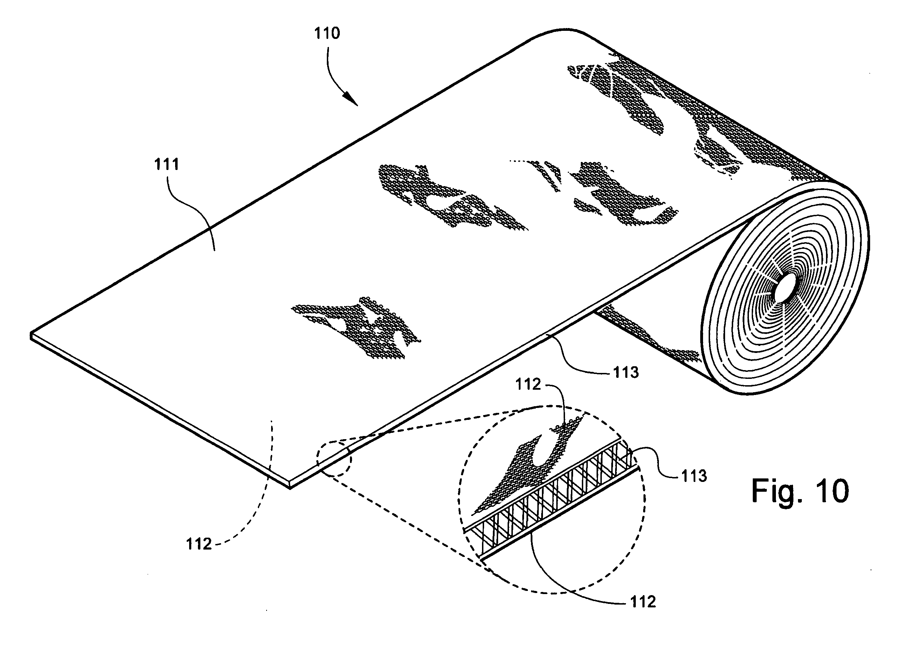

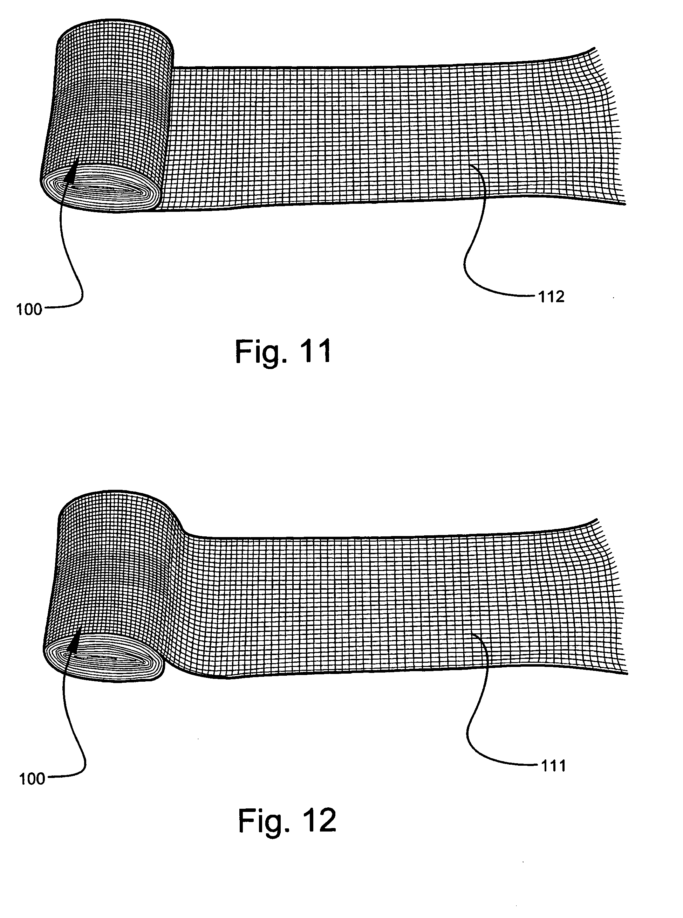

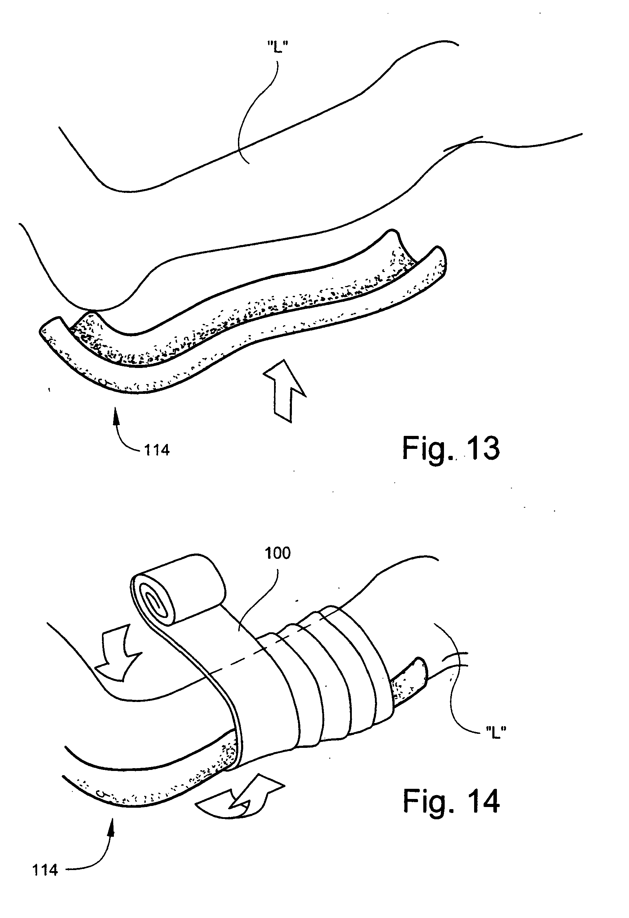

A water-resistant elasticized retention bandage according to a preferred embodiment of the invention is illustrated in Figures 10-13, and shown generally at reference numeral 100. As shown in Figure 10, the retention bandage 10 comprises an elongate knitted fabric having two opposing surfaces 111,112 and an intermediate spacer section 113. The spacer 113 both separates and connects the opposed surfaces 111, 112. The elastic retention bandage 100 is constructed at least in part of hydrophobic, water-resistant monofilament yarns to create an efficient breathable, porous, lightweight structure that can dry rapidly. The retention bandage 100 can be stretched sufficiently in both width and length directions to provide excellent application aspects that avoids creasing and wrinkling when applied to an anatomical shape. As shown in Figures 13 and 14, the retention bandage 100 can be applied to an anatomical shape of a patient such as a leg "L" to hold in position an orthopedic device such as a splint 114.

Preferably, the retention bandage 100 can be stretched to a greater extent in the length direction than in the width direction in order to give more compressive force to hold the splint 114, or other medical device such as a swab or padding, in position without slippage.

An adhesive such as a hot melt adhesive can be applied to one surface of the bandage substrate to tack the material in position and prevent movement of the bandage 100. The adhesive permits adhesion of the bandage 100 to itself and prevents movement of the bandage 100 when in situ supporting or holding in position the splint or padding or swab. The adhesive mass is preferably based on a weight of between about 2 and about 30 grams per square meter, and is preferably of a type that is skin friendly. Alternatively, the bandage 10 can be used without an adhesive, and the end of the bandage 10 can be secured by a clip, pin, sticky tape, hook and loop fasteners, and combinations thereof.

The knitted spacer fabric 113 is constructed of highly resilient monofilament yarns combined with elastic yarns that give a stretch capability to the structure to aid and support application to the anatomical structure. The use of the monofilament yarns ensures effective water drainage and drying due to very low surface area and surface energy. The yarns used in the elastic retention bandage 100 are polypropylene, polyester, nylon, polyurethane and combinations thereof, and the fabric of the bandage 100 can be formed by weaving, knitting or other suitable method.

The retention bandage 100 is preferably constructed using a pillar, inlay, and 'V' needle structure in the center stitch. The bandage 100 is a lightweight structure and preferably weighs between about 50 and about 200 grams per square meter. The invention can provide a more comfortable, conformable, breathable, water-resistant bandage 100 at a lower cost than other products available in the market. The present invention's use of a novel construction of monofilament yarns yields excellent padding and openness that permits safer application and air circulation around the injury site. The substrate has a relatively high moisture vapor transmission rate of about 1500 grams per day per square meter.

The invention can significantly improve air circulation leading to improvements in skin condition and maceration levels. The present invention allows the patient to swim, bath or shower and keep the injury site clean and healthy without damaging the support or retention bandage.

A water-resistant elasticized retention bandage and a water-resistant undercast liner are described above. Various details of the invention may be changed without departing from its scope. Furthermore, the foregoing description of the preferred embodiments of the invention and the best mode for practicing the invention are provided for the purpose of illustration only and not for the purpose of limitation.

## Claims

1. A fabric substrate (10, 30, 100) suitable for lining between a cast and a person comprising:
(A) a pair of opposing faces (11, 12; 31, 32; 111, 112) formed by pillar and inlay stitches, wherein the fabric is constructed at least in part of hydrophobic, water-resistant yarns; and
(B) an intermediate tying layer (13, 33, 113) separating and interconnecting the opposing faces, wherein the tying layer is constructed of elastic yarns combined with highly resilient monofilament yarns selected from the group consisting of nylon, polypropylene, polyester, polyurethane and combinations thereof wherein the elastic and monofilament yarns of the tying layer are stitched in a V stitch pattern such that the elastic yarns extend between one opposing fabric face and the other face.

2. The substrate according to claim 1, further including an adhesive coating on the opposing faces.

3. The substrate according to Claim 2, wherein the adhesive is a low tack pressure sensitive adhesive selected from the group consisting of acrylic adhesives, silicone adhesives, and combinations thereof.

4. The substrate according to Claim 1, wherein the V stitch pattern is a 3 needle V stitch pattern.

5. The substrate according to Claim 1, wherein the V stitch pattern is a 5 needle V stitch pattern.

6. The substrate according to Claim 1, wherein the substrate is formed such that it has at least 50 courses per meter and weighs between 50 and 400 grams per square meter.

7. The substrate according to claim 6, wherein the substrate weighs between 100 and 250 grams per square meter and has a nominal thickness when not compressed or under tension of between 1.5 and 3.5 mm.

## Patentansprüche

1. Stoffsubstrat (10, 30, 100), das als Auskleidung zwischen einem Gips und einer Person geeignet ist, umfassend:
(A) ein Paar gegenüberliegender Seiten (11, 12; 31, 32; 111, 112), die durch Franse-Schuss-Stiche geformt sind, worin der Stoff zumindest teilweise aus hydrophoben, wasserfesten Garnen hergestellt ist; und
(B) eine dazwischen liegende Anknüpfungsschicht (13, 33, 113), die die gegenüberliegenden Seiten voneinander trennt und miteinander verbindet, worin die Anknüpfungsschicht aus elastischen Garnen in Kombination mit äußerst nachgiebigen monofilen Garnen, ausgewählt aus der Gruppe von Nylon, Polypropylen, Polyester, Polyurethan und Kombinationen davon, hergestellt ist, worin die elastischen und monofilen Garne der Anknüpfungsschicht in einem V-Stichmuster genäht sind, so sich die elastischen Garne zwischen einer gegenüberliegenden Stoffseite und der anderen Seite erstrecken.

2. Substrat nach Anspruch 1, das ferner eine Klebebeschichtung auf den gegenüberliegenden Seiten aufweist.

3. Substrat nach Anspruch 2, worin der Klebestoff ein weniger haftender Kontaktkleber ist, der aus der aus Acryl-Klebstoffe, Silikon-Klebstoffe und Kombinationen davon bestehenden Gruppe ausgewählt ist.

4. Substrat nach Anspruch 1, worin das V-Stichmuster ein 3-Nadel-V-Stichmuster ist.

5. Substrat nach Anspruch 1, worin das V-Stichmuster ein 5-Nadel-V-Stichmuster ist.

6. Substrat nach Anspruch 1, worin das Substrat so geformt ist, dass es zumindest 50 Läufe pro Meter aufweist und zwischen 50 und 400 Gramm pro Quadratmeter wiegt.

7. Substrat nach Anspruch 6, worin das Substrat zwischen 100 und 250 Gramm pro Quadratmeter wiegt und eine Nenndicke im nicht komprimierten oder nicht gespannten Zustand zwischen 1,5 und 3,5 mm aufweist.

## Revendications

1. Substrat en tissu (10, 30, 100) convenant pour une utilisation comme doublure entre un plâtre et une personne, comprenant :
(A) une paire de faces opposées (11, 12 ; 31, 32 ; 111, 112) formées de chaînette tramée, le tissu étant constitué au moins en partie de fils hydrophobes, résistant à l'eau ; et
(B) une couche de liaison intermédiaire (13, 33, 113) séparant et reliant l'une à l'autre les faces opposées, la couche de liaison étant constituée de fils élastiques combinés avec des fils monofilament hautement résilients sélectionnés dans le groupe constitué du nylon, du polypropylène, du polyester, du polyuréthane et de combinaisons de ceux-ci, les fils élastiques et les fils monofilament de la couche de liaison étant liés par un point en V de telle sorte que les fils élastiques s'étendent entre une des faces opposées du tissu et l'autre face.

2. Substrat selon la revendication 1, comprenant en outre un revêtement adhésif sur les faces opposées.

3. Substrat selon la revendication 2, dans lequel l'adhésif est un adhésif sensible à la pression à faible adhésivité instantanée sélectionné dans le groupe constitué d'adhésifs acryliques, d'adhésifs de silicone, et de combinaisons de ceux-ci.

4. Substrat selon la revendication 1, dans lequel le point en V est un point en V 3 aiguilles.

5. Substrat selon la revendication 1, dans lequel le point en V est un point en V 5 aiguilles.

6. Substrat selon la revendication 1, le substrat étant formé de telle sorte qu'il comporte au moins 50 rangées de mailles par mètre et pèse de 50 g/m² à 400 g_{/}m²_{.}

7. Substrat selon la revendication 6, le substrat pesant de 100 g/m² à 250 g/m² et ayant une épaisseur nominale de 1,5 mm à 3,5 mm quand il n'est pas comprimé ou sous tension.
